# EUROPÄISCHE PATENTANMELDUNG

(11) **EP 4 631 575 A2**
(43) Veröffentlichungstag der Anmeldung: **15.10.2025**
(21) Anmeldenummer: 25185230.7
(22) Anmeldetag: 20.12.2024
(51) Int. Cl.: A61Q 7/00

(54) **ZUSAMMENSETZUNG UMFASSEND PHOSPHINSÄURE UND DIMETHYLGLYCIN**

(30) Priorität: 22.12.2023 DE 102023005341
(62) Teilanmeldung aus: 24837909.1
(71) Anmelder: Dr. Kurt Wolff GmbH & Co. KG, 33611 Bielefeld (DE)
(72) Erfinder: SCHULZE ZUR WIESCHE, Erik, 33611 Bielefeld (DE); VÖLKER, Jörn Michael, 33611 Bielefeld (DE)
(74) Vertreter: Weickmann & Weickmann PartmbB

(57) **Zusammenfassung**

Die vorliegende Erfindung betrifft eine Zusammensetzung umfassend Phosphinsäure oder ein Salz davon und Dimethylglycin oder ein Salz davon, insbesondere in einer Darreichungsform zur oralen oder topischen Verabreichung. Zudem betrifft die vorliegende Erfindung die (kosmetische und/oder medizinische) Verwendung dieser Zusammensetzung zur Verbesserung des Zustands der Haare und/oder der Haut, zur Förderung des Stoffwechsels der Haare und/oder der Haut, zur Förderung des Haarwachstums und/oder zur Behandlung und/oder Vorbeugung von Haarausfall. Weiterhin betrifft die vorliegende Erfindung ein Nahrungsergänzungsmittel zur Förderung des Stoffwechsels der Haare und/oder der Haut und/oder zur Förderung des Haarwachstums und/oder zur Behandlung und/oder Vorbeugung von Haarausfall. Ferner betrifft die Erfindung einen Kit umfassend Dimethylglycin und/oder ein Salz von Dimethylglycin sowie Phosphinsäure und/oder wenigstens ein Salz der Phosphinsäure, wobei die beiden Verbindungen in getrennten Verabreichungsformen vorliegen.

## Beschreibung

Die vorliegende Erfindung betrifft eine Zusammensetzung umfassend Phosphinsäure oder ein Salz davon und Dimethylglycin oder ein Salz davon, insbesondere in einer Darreichungsform zur oralen oder topischen Verabreichung. Zudem betrifft die vorliegende Erfindung die (kosmetische und/oder medizinische) Verwendung dieser Zusammensetzung zur Verbesserung des Zustands der Haare und/oder der Haut, zur Förderung des Stoffwechsels der Haare und/oder der Haut, zur Förderung des Haarwachstums und/oder zur Behandlung und/oder Vorbeugung von Haarausfall. Weiterhin betrifft die vorliegende Erfindung ein Nahrungsergänzungsmittel zur Förderung des Stoffwechsels der Haare und/oder der Haut und/oder zur Förderung des Haarwachstums und/oder zur Behandlung und/oder Vorbeugung von Haarausfall.

Das menschliche Haar hat seine Bedeutung als Schutzfunktion für den Körper mittlerweile zu einem großen Teil verloren. Allerdings hat gesundes Haar weltweit eine große kulturelle Bedeutung für Frauen und Männer und wird häufig als Zeichen von Wohlstand und Gesundheit angesehen. Folglich beeinflusst beispielsweise durch Haarausfall bedingtes schütteres Haar oft die Lebensqualität auf negative Weise.

Dabei sind die Ursachen von Haarausfall vielfältig und reichen von altersbedingtem Haarausfall über genetisch bzw. hormonell bedingte Störungen, Autoimmunstörungen, Stoffwechselstörungen, einschließlich Fehlernährung, bis hin zu Infektionen, Traumen, Tumoren und Medikamenten wie Zytostatika und Antikoagulantien.

Ein durch Hormone wie Androgene hervorgerufener Haarausfall wird als androgenetische Alopezie (Alopecia androgenetica oder auch AGA) bezeichnet und gilt insbesondere bei Männern aber ebenfalls auch bei Frauen als die häufigste Ursache für Haarausfall. Hierbei kann zwischen männlichen und weiblichen Haarausfallmustern unterschieden werden. Andere häufige Formen von nicht vernarbender Alopezie umfassen Telogenes Effluvium (TE), einen diffusen Haarausfall, sowie den akut einsetzenden entzündlich bedingten kreisrunden Haarausfall, der auch als Alopecia areata (AA) bezeichnet wird.

Der Einfluss von Nährstoffmangel auf die Haarstruktur und das Haarwachstum sind durchaus erheblich. Zu den Auswirkungen auf das Haarwachstum gehören das akute Telogene Effluvium (TE), eine bekannte Folge von plötzlichem Gewichtsverlust oder verminderter Proteinzufuhr, sowie die diffuse Alopezie, die bei Niacinmangel auftritt. In Studien wurde auch über mögliche Zusammenhänge zwischen Nährstoffmangel und chronischer TE, androgenetischer Alopezie (AGA), weiblichem Haarausfall (FPHL) und Alopecia areata (AA) berichtet (Guo E.L., Katta R. Diet and hair loss: effects of nutrient deficiency and supplement use. Dermatol Pract Concept. 2017;7(1):1).

Mikronährstoffe wie Vitamine und Mineralstoffe spielen eine wichtige, aber nicht ganz eindeutige Rolle für die normale Entwicklung der Haarfollikel und die Funktion der Immunzellen. Dementsprechend gibt es in zahlreichen Studien Hinweise auf einen Zusammenhang zwischen Haarausfall und Mikronährstoffmangel.

Einen Überblick über die Rolle von Vitaminen und Mineralstoffen wie Vitamin A, Vitamin B, Vitamin C, Vitamin D, Vitamin E, Eisen, Selen und Zink, bei nicht vernarbender Alopezie geben Almohanna et al. (Almohanna, Hind M et al. "The Role of Vitamins and Minerals in Hair Loss: A Review." Dermatology and therapy vol. 9,1 (2019): 51-70).

Guo und Katta beschreiben die Studienlage im Hinblick auf einen Mangel von bzw. Supplementation mit Eisen, Zink, Niacin, Fettsäuren, Selen, Vitamin D, Vitamin A, Vitamin E, Folsäure, Biotin, Aminosäuren und Proteinen sowie Antioxidantien.

In ähnlicher Weise befasst sich ein Übersichtsartikel von Goldberg und Lenzy mit Veränderungen des Haares aufgrund von allgemeiner Fehlernährung und Mangel an Mikronährstoffen in bestimmten Patientengruppen (Goldberg L.J., Lenzy Y. Nutrition and hair. Clin Dermatol. 2010 Jul-Aug;28(4):412-9).

Eine mögliche Rolle von Nahrungsergänzungsmitteln wie etwa Aminosäuren, Vitaminen und Spurenelementen, essentiellen Fettsäuren, den Coenzymen Q und A, Bioflavonoiden, Inositol, Methylsulfonylmethan (MSM), Dimethylglycin (DMG), Kieselsäure, Kelp, Grüner Tee-Extrakt, Sägepalme-Extrakt und Weintraubensamenextrakt bei der Vorbeugung von Haarausfall wird schließlich in einem Übersichtsartikel von Jain et al. beschrieben (Jain, Pushpendra & Joshi, Himanshu & Das, Debajyoti. (2012). Drug that Causes Hair Loss and Promotes Hair Growth-A Review. International Journal of Research in Pharmaceutical and Biomedical Sciences. 3. 1476-82).

Ein Mangel an solchen Mikronährstoffen könnte ein veränderbarer Risikofaktor für die Entwicklung, Prävention und Behandlung von Alopezie sein.

Während Nahrungsergänzungsmittel mit Vitaminen und Mineralstoffen relativ preiswert und leicht zugänglich sind, setzt deren Einsatz jedoch voraus, zu wissen, welche Vitamine und Mineralien bei der Behandlung von Haarausfall hilfreich sind. Bis zum heutigen Tag sind jedoch nur wenige Studien durchgeführt worden, die die Wirkung einer Mikronährstoffsupplementierung auf das Haarwachstum bei Patienten mit Mikronährstoffmangel und Alopezie untersuchen, um einen Zusammenhang zwischen Haarausfall und Mikronährstoffmangel festzustellen, und es gibt noch viel weniger Literatur über die Auswirkungen von Supplementierung bei Personen ohne Nährstoffmangel.

Zudem gibt es Hinweise, dass eine Überdosierung bestimmter Mikronährstoffe, wie beispielsweise Vitamin A, Vitamin E oder Selen, Haarausfall sogar begünstigen kann (Almohanna et al.; Guo and Katta).

Zusammenfassend können sich Nahrungsergänzungsmittel sowohl positiv als auch negativ auf Haarausfall auswirken, und, sofern kein Nährstoffmangel festgestellt wird, gibt es nur wenig Hinweise darauf, welche Art von Supplementierung geeignet ist, um das Haarwachstum wiederherzustellen oder weiteren Haarausfall zu verhindern.

Daneben gibt es bis heute nur wenige zugelassene pharmazeutische Behandlungsmöglichkeiten für Haarausfall, wie androgenetische Alopezie, und die bereits vorhandenen Behandlungsmöglichkeiten sind entweder nicht ausreichend wirksam oder gehen mit unangenehmen Nebenwirkungen einher. Ähnlich unerfreulich sieht es beispielsweise bei Behandlungsmöglichkeiten für den akut einsetzenden entzündlich bedingten kreisrunden Haarausfall Alopecia areata, und für durch andere Ursachen bedingten Haarausfall aus.

Damit besteht weiterhin ein Bedarf an verbesserten Behandlungsmethoden, insbesondere ein Bedarf an Zusammensetzungen, welche oral und/oder topisch gegen Haarausfall eingesetzt werden können, wobei diese Zusammensetzungen keine oder nur vernachlässigbare Nebenwirkungen zeigen sollen. Insbesondere besteht ein Bedarf an Zusammensetzungen in Form von Nahrungsergänzungsmitteln, die leicht erhältlich sind und unkompliziert angewendet werden können. Ebenso besteht ein Bedarf an Zusammensetzungen, welche pharmazeutischer Natur sind und keine Nebenwirkungen zeigen oder welche kosmetischer Natur sind.

Ausgehend hiervon war es die Aufgabe der vorliegenden Erfindung, eine gut verträgliche Zusammensetzung zur Behandlung von Haaren und der Haut, insbesondere zur Prävention und/oder Behandlung von Haarausfall, bereitzustellen. Weiterhin sollte sich diese Zusammensetzung oral und/oder topisch anwenden lassen und die Nachteile der aus dem Stand der Technik bekannten Zusammensetzungen überwinden.

Erfindungsgemäß wird die Aufgabe durch die Bereitstellung einer Zusammensetzung gelöst, welche
a) Phosphinsäure und/oder wenigstens ein Salz der Phosphinsäure, und
b) Dimethylglycin und/oder ein Salz von Dimethylglycin umfasst.

Phosphinsäure, auch Hypophosphorige Säure, ist eine der Säuren des Phosphors und hat die Summenformel H₃PO₂. Ihre Salze werden Phosphinate oder Hypophosphite genannt. Einwertige und/oder zweiwertige Salze der Phosphinsäure sind erfindungsgemäß bevorzugt. Ein bevorzugtes einwertiges Salz ist Natriumhypophosphit (auch Natriumphosphinat) (Na(H₂PO₂)). Insbesondere bevorzugt sind zweiwertige Salze der Phosphinsäure, wobei Calciumhypophosphit (Ca(H₂PO₂)₂) wiederum besonders bevorzugt ist.

Bevorzugt enthält die erfindungsgemäße Zusammensetzung Hypophosphit und insbesondere Calciumhypophosphit in einer Menge von 0,0001 bis 35,0 Gewichtsprozent (Gew.-%), bevorzugt von 0,001 bis 25,0 Gew.-%, jeweils bezogen auf das Gesamtgewicht der Zusammensetzung.

In einer bevorzugten Ausführungsform ist die erfindungsgemäße Zusammensetzung zur oralen Anwendung ausgestaltet und enthält Hypophosphit und insbesondere Calciumhypophosphit in einer Menge von 0,0001 bis 35,0 Gewichtsprozent, bevorzugt 1,0 bis 25,0 Gew.-%, stärker bevorzugt 5,0 bis 25,0 Gew.-%, jeweils bezogen auf das Gesamtgewicht der Zusammensetzung.

In einer alternativen Ausführungsform ist die erfindungsgemäße Zusammensetzung zur topischen Anwendung ausgestaltet und enthält Hypophosphit und insbesondere Calciumhypophosphit in einer Menge von 0,001 bis 25,0 Gew.-%, bevorzugt 0,01 bis 15,0 Gew.-%, stärker bevorzugt 0,1 bis 10,0 Gew.-%, besonders bevorzugt 0,1 bis 5,0 Gew.-%, jeweils bezogen auf das Gesamtgewicht der Zusammensetzung.

In einer bevorzugten Ausführungsform der Erfindung kann die erfindungsgemäße Zusammensetzung 0,0001 Gew.-%, 0,001 Gew.-%, 0,01 Gew.-%, 0,1 Gew.-%, 1 Gew.-%, 2 Gew.-%, 3 Gew.-%, 4 Gew.-%, 5 Gew.-%, 6 Gew.-%, 7 Gew.-%, 8 Gew.-%, 9 Gew.-%, 10 Gew.-%, 11 Gew.-%, 12 Gew.-%, 13 Gew.-%, 14 Gew.-%, 15 Gew.-%, 20 Gew.-%, 25 Gew.-% oder 35 Gew.-% Phosphinsäure und/oder ein Salz von Phosphinsäure enthalten, jeweils bezogen auf das Gesamtgewicht der Zusammensetzung.

Es hat sich überraschend gezeigt, dass die erfindungsgemäße Zusammensetzung mit Dimethylglycin und/oder einem Salz davon sowie Phosphinsäure und/oder einem Salz davon eine ausgezeichnete Wirksamkeit in der Behandlung von Haarausfall, insbesondere von erblich bedingtem und altersbedingtem Haarausfall, aufweist. Zudem ist die Zusammensetzung medizinisch besonders verträglich.

Dimethylglycin (N,N-Dimethylglycin) kommt in Pflanzen, Tieren und dem Menschen vor, wobei es im Menschen nur in sehr geringen Mengen gebildet wird. Es entsteht bei einer mehrstufigen Biosynthese von Glycin aus Cholin als Zwischenprodukt durch Transaminierung von Betain mit Betain-Homocystein-Methylase.

N,N-Dimethylglycin, auch (Dimethylamino)essigsäure, wird durch die folgende chemische Formel 1 dargestellt:

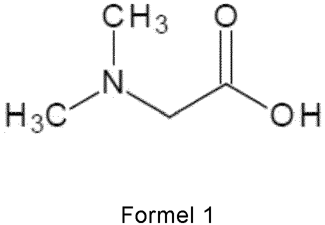

Gemäß der vorliegenden Erfindung wurde überraschenderweise gefunden, dass sich Dimethylglycin und/oder ein Salz von Dimethylglycin nicht nur zur topischen Anwendung eignet, sondern in Kombination mit Phospinsäure und/oder wenigstens einem Salz der Phosphinsäure als orales Präparat in hervorragender Weise zur Verbesserung des Zustands der Haare und der Haut, insbesondere zur Behandlung oder Vorbeugung von Haarausfall geeignet ist.

Erfindungsgemäß ist nicht nur der Einsatz von Dimethylglycin, sondern auch der von dessen Salzen, Solvaten und Hydraten. Dabei handelt es sich bevorzugt um pharmazeutisch bzw. kosmetisch annehmbare Salze von Dimethylglycin. Das Salz ist besonders bevorzugt ein wasserlösliches Salz mit einer Löslichkeit in Wasser von mindestens 10 g/l bei 20°C.

In einer bevorzugten Aufführungsform ist das Salz von Dimethylglycin ein Alkali-, Erdalkali- oder Ammoniumsalz von Dimethylglycin.

Beispiele sind Natrium-, Kalium-, Calcium-, Magnesium- und Ammoniumsalze. Bei den Ammoniumsalzen trägt das Ammoniumkation eine bis vier Alkylgruppen mit jeweils unabhängig voneinander 1 bis 4 Kohlenstoffatomen. Bevorzugt sind das Natrium- und Kaliumsalz von Dimethylglycin, insbesondere das Natriumsalz von Dimethylglycin, nämlich Natrium-dimethylglycinat (Natrium-N,N-dimethylglycinat).

In einer alternativ bevorzugten Ausführungsform kann das Salz von Dimethylglycin das Salz einer anorganischen Säure mit Dimethylglycin sein.

Beispiele für Salze von Dimethylglycin mit einer anorganischen Säure sind das Hydrochlorid, Hydrobromid, Hydroiodid, Hydrogensulfat, Sulfat, Hydrogensulfit, Sulfit, Hydrogencarbonat, Carbonat, Monophosphat, Diphosphat und Triphosphat von Dimethylglycin sowie Mischungen daraus. Insbesondere bevorzugt ist das Hydrochlorid von Dimethylglycin, nämlich Dimethylglycin-hydrochlorid (N,N-Dimethylglycin-hydrochlorid).

Erfindungsgemäß bevorzugt ist das Dimethylglycin und/oder Salz von Dimethylglycin ausgewählt aus der Gruppe bestehend aus Dimethylglycin, Natrium-dimethylglycinat und Dimethylglycin-hydrochlorid.

Erfindungsgemäß bevorzugt ist das Dimethylglycin und/oder Salz von Dimethylglycin ausgewählt aus der Gruppe bestehend aus Dimethylglycin, Natrium-dimethylglycinat und Dimethylglycin-hydrochlorid.

Es wird angenommen, dass die (Kopf)Haut gepflegt und die Hautbarriere gestärkt wird. Es erzielt so erfindungsgemäß eine deutliche haarwurzel- und (kopf)hautstärkende Wirkung, insbesondere bei der Behandlung von alltags- bzw. altersbedingt gestresster oder geschwächter Haut sowie Haarausfall, wie dem erblich bedingten und altersbedingten Haarausfall.

Die erfindungsgemäße Zusammensetzung enthält Dimethylglycin und/oder ein Salz von Dimethylglycin vorzugsweise in einem Anteil von 0,0001 bis 35,0 Gewichtsprozent (Gew.-%), bevorzugt von 0,001 bis 25,0 Gew.-%, jeweils bezogen auf das Gesamtgewicht der Zusammensetzung.

In einer bevorzugten Ausführungsform ist die erfindungsgemäße Zusammensetzung zur oralen Anwendung ausgestaltet und enthält Dimethylglycin und/oder ein Salz von Dimethylglycin in einer Menge von 0,0001 bis 35,0 Gew.-%, bevorzugt 3,0 bis 32,0 Gew.-%, jeweils bezogen auf das Gesamtgewicht der Zusammensetzung.

In einer alternativen Ausführungsform ist die erfindungsgemäße Zusammensetzung zur topischen Anwendung ausgestaltet und enthält Dimethylglycin und/oder ein Salz von Dimethylglycin in einer Menge von 0,001 bis 25,0 Gew.-%, bevorzugt 0,01 bis 15,0 Gew.-%, stärker bevorzugt 0,1 bis 10,0 Gew.-%, besonders bevorzugt 0,1 bis 5,0 Gew.-%, jeweils bezogen auf das Gesamtgewicht der Zusammensetzung.

In einer bevorzugten Ausführungsform der Erfindung kann die erfindungsgemäße Zusammensetzung 0,0001 Gew.-%, 0,001 Gew.-%, 0,01 Gew.-%, 0,1 Gew.-%, 1 Gew.-%, 2 Gew.-%, 3 Gew.-%, 4 Gew.-%, 5 Gew.-%, 6 Gew.-%, 7 Gew.-%, 8 Gew.-%, 9 Gew.-%, 10 Gew.-%, 11 Gew.-%, 12 Gew.-%, 13 Gew.-%, 14 Gew.-%, 15 Gew.-%, 20 Gew.-% 25 Gew.-% oder 35 Gew.-% Dimethylglycin und/oder ein Salz von Dimethylglycin enthalten, jeweils bezogen auf das Gesamtgewicht der Zusammensetzung.

Bevorzugt enthält die erfindungsgemäße Zusammensetzung Dimethylglycin und/oder ein Salz von Dimethylglycin als chemischen Reinstoff, einschließlich der jeweiligen Solvate und Hydrate (z.B. des Dihydrats von Natriumdimethylglycinat), da so die Reinheit der Zusammensetzung erhöht und das Auftreten von unerwünschten Nebenwirkungen vermindert werden kann.

Die erfindungsgemäße Zusammensetzung enthält Dimethylglycin und/oder ein Salz davon sowie Phospinsäure und/oder ein Salz davon vorzugsweise in einem molaren Verhältnis von 1:10 bis 10:1, bevorzugt 1:5 bis 5:1, besonders bevorzugt 1:2 bis 2:1.

In einer bevorzugten Ausführungsform enthält die erfindungsgemäße Zusammensetzung mindestens einen weiteren Wirkstoff c), wobei der mindestens eine weitere Wirkstoff ausgewählt ist aus Amla-Extrakt, β-Carotin, Biotin, Carnitin, Curcumin, Echinacea, Ectoin, Eisen, Folsäure, Granatapfel-Extrakt, Grüntee-Extrakt, Hirseextrakt, Hirseöl, Kürbiskernöl, Kreatin, L-Cystein, L-Lysin, Niacin, Niacinamid, Pantolacton, Panthothensäure, Piperin, Procyanidin, Rosmarin-Extrakt, Sägepalme-Extrakt, Schachtelhalm-Extrakt, Selen, Silicium, Taurin, Tocopherylacetat, Tomatenextrakt, Ubichinon-10, Vitamin B1, Vitamin B2, Vitamin B6, Vitamin B12, Vitamin C, Vitamin E, Zink und Kombinationen hiervon, insbesondere ausgewählt aus Zink, Biotin, Hirseextrakt, Selen und Kombinationen hiervon.

Amla-Extrakt ist ein Extrakt aus der Indischen Stachelbeere (Phyllanthus emblica). In der erfindungsgemäßen Zusammensetzung kann Amla-Extrakt in einer Menge von 0,1-50,0 Gew.-%, bevorzugt 1-30 Gew.-%, stärker bevorzugt 5-25 Gew.-% enthalten sein, jeweils bezogen auf das Gesamtgewicht der Zusammensetzung.

β-Carotin ist eine Vorstufe von Vitamin A und wird deshalb auch als Provitamin A bezeichnet. In der erfindungsgemäßen Zusammensetzung kann β-Carotin in einer Menge von 0,01-1,0 Gew.-%, bevorzugt 0,05-0,5 Gew.-% enthalten sein, jeweils bezogen auf das Gesamtgewicht der Zusammensetzung.

Biotin, welches auch als Vitamin B7 oder Vitamin H bezeichnet wird, ist ein wasserlösliches Vitamin aus dem B-Komplex. Biotin kann erfindungsgemäß Haarausfall weiter vermindern und die Haut stärken. In der erfindungsgemäßen Zusammensetzung kann Biotin in einer Menge von 0,001-10,0 Gew.-%, bevorzugt 0,005-1,0 Gew.-% enthalten sein, jeweils bezogen auf das Gesamtgewicht der Zusammensetzung.

In der erfindungsgemäßen Zusammensetzung kann Carnitin in einer Menge von 0,001 Gew.-% bis 10,0 Gew.-%, bevorzugt 0,005 Gew.-% bis 7,50 Gew.-% enthalten sein, jeweils bezogen auf das Gesamtgewicht der Zusammensetzung.

In der erfindungsgemäßen Zusammensetzung kann Curcumin in einer Menge von 0,1-20,0 Gew.-%, bevorzugt 1-15 Gew.-% enthalten sein, jeweils bezogen auf das Gesamtgewicht der Zusammensetzung.

Echinacea kann die Blutzirkulation der Kopfhaut anregen und so die Haarfollikel mit sauerstoff- und nährstoffreichem Blut versorgen, was sich weiter stabilisierend auf die Haare und insbesondere die Haarwurzel auswirkt. In der erfindungsgemäßen Zusammensetzung kann Echinacea in einer Menge von 0,001 Gew.-% bis 10,0 Gew.-%, bevorzugt 0,005 Gew.-% bis 7,50 Gew.-% enthalten sein, jeweils bezogen auf das Gesamtgewicht der Zusammensetzung.

Ectoin ist eine zyklische Aminosäure und stabilisiert erfindungsgemäß die natürliche Struktur von Haaren weiter. In der erfindungsgemäßen Zusammensetzung kann Ectoin in einer Menge von 0,001 Gew.-% bis 10,0 Gew.-%, bevorzugt 0,005 Gew.-% bis 7,50 Gew.-% enthalten sein, jeweils bezogen auf das Gesamtgewicht der Zusammensetzung.

In der erfindungsgemäßen Zusammensetzung kann Eisen in einer Menge von 0,01-1,0 Gew.-%, bevorzugt 0,05-0,5 Gew.-% enthalten sein, jeweils bezogen auf das Gesamtgewicht der Zusammensetzung.

In der erfindungsgemäßen Zusammensetzung kann Folsäure in einer Menge von 0,001-1,0 Gew.-%, bevorzugt 0,05-0,5 Gew.-% enthalten sein, jeweils bezogen auf das Gesamtgewicht der Zusammensetzung.

In der erfindungsgemäßen Zusammensetzung kann Granatapfel-Extrakt in einer Menge von 0,1-20,0 Gew.-%, bevorzugt 1-15 Gew.-% enthalten sein, jeweils bezogen auf das Gesamtgewicht der Zusammensetzung.

In der erfindungsgemäßen Zusammensetzung kann Grüner Tee-Extrakt in einer Menge von 0,1-10,0 Gew.-%, bevorzugt 1,0-5,0 Gew.-%, jeweils bezogen auf das Gesamtgewicht der Zusammensetzung.

In der erfindungsgemäßen Zusammensetzung kann Hirseextrakt in einer Menge von 0,01-40,0 Gew.-%, bevorzugt 1-35 Gew.-%, stärker bevorzugt 5-30 Gew.-% enthalten sein, jeweils bezogen auf das Gesamtgewicht der Zusammensetzung.

In der erfindungsgemäßen Zusammensetzung kann Hirseöl in einer Menge von 0,01-30,0 Gew.-%, bevorzugt 1-15 Gew.-% enthalten sein, jeweils bezogen auf das Gesamtgewicht der Zusammensetzung.

In der erfindungsgemäßen Zusammensetzung kann Kürbiskernöl in einer Menge von 0,1-20,0 Gew.-%, bevorzugt 1-15 Gew.-% enthalten sein, jeweils bezogen auf das Gesamtgewicht der Zusammensetzung.

In der erfindungsgemäßen Zusammensetzung kann Kreatin 0,001 Gew.-% bis 10,0 Gew.-%, bevorzugt 0,005 Gew.-% bis 7,50 Gew.-% enthalten sein, jeweils bezogen auf das Gesamtgewicht der Zusammensetzung.

In der erfindungsgemäßen Zusammensetzung kann L-Cystein in einer Menge von 0,01-20,0 Gew.-%, bevorzugt 0,1-1,0 Gew.-% enthalten sein, jeweils bezogen auf das Gesamtgewicht der Zusammensetzung.

In der erfindungsgemäßen Zusammensetzung kann L-Lysin in einer Menge von 0,1-20,0 Gew.-%, bevorzugt 1-15 Gew.-% enthalten sein, jeweils bezogen auf das Gesamtgewicht der Zusammensetzung.

In der erfindungsgemäßen Zusammensetzung kann Niacin in einer Menge von 0,1-10,0 Gew.-%, bevorzugt 0,5-5,0 Gew.-% enthalten sein, jeweils bezogen auf das Gesamtgewicht der Zusammensetzung.

Niacinamid (auch Nicotinamid) ist das Amid der Nicotinsäure und wird auch als Vitamin B3 bezeichnet. Neben anderen Eigenschaften wie beispielweise einer Verringerung von oxidativem Stress hat das Niacinamid erfindungsgemäß eine das Haarwachstum stimulierende Wirkung. In der erfindungsgemäßen Zusammensetzung kann Niacinamid 0,001 Gew.-% bis 10,0 Gew.-%, bevorzugt 0,005 Gew.-% bis 7,50 Gew.-% enthalten sein, jeweils bezogen auf das Gesamtgewicht der Zusammensetzung.

Pantolacton entstammt der Gruppe der substituierten Lactone und regt erfindungsgemäß die Wachstumsfaktoren der Haarwurzeln weiter an. In der erfindungsgemäßen Zusammensetzung kann Pantolacton 0,001 Gew.-% bis 10,0 Gew.-%, bevorzugt 0,005 Gew.-% bis 7,50 Gew.-% enthalten sein, jeweils bezogen auf das Gesamtgewicht der Zusammensetzung.

In der erfindungsgemäßen Zusammensetzung kann Panthothensäure in einer Menge von 0,01-10,0 Gew.-%, bevorzugt 0,1-2,0 Gew.-% enthalten sein, jeweils bezogen auf das Gesamtgewicht der Zusammensetzung.

In der erfindungsgemäßen Zusammensetzung kann Piperin in einer Menge von 0,01-5,0 Gew.-%, bevorzugt 0,05-2,0 Gew.-% enthalten sein, jeweils bezogen auf das Gesamtgewicht der Zusammensetzung.

In der erfindungsgemäßen Zusammensetzung kann Procyanidin in einer Menge von 0,1-20,0 Gew.-%, bevorzugt 1-15 Gew.-% enthalten sein, jeweils bezogen auf das Gesamtgewicht der Zusammensetzung.

In der erfindungsgemäßen Zusammensetzung kann Rosmarin-Extrakt in einer Menge von 0,001-20,0 Gew.-%, bevorzugt 0,01-5,0 Gew.-% enthalten sein, jeweils bezogen auf das Gesamtgewicht der Zusammensetzung.

In der erfindungsgemäßen Zusammensetzung kann Sägepalme-Extrakt in einer Menge von 0,1-20,0 Gew.-%, bevorzugt 1-15 Gew.-% enthalten sein, jeweils bezogen auf das Gesamtgewicht der Zusammensetzung.

In der erfindungsgemäßen Zusammensetzung kann Schachtelhalm-Extrakt in einer Menge von 0,01-1,0 Gew.-%, bevorzugt 0,1-1,0 Gew.-% enthalten sein, jeweils bezogen auf das Gesamtgewicht der Zusammensetzung.

In der erfindungsgemäßen Zusammensetzung kann Selen in einer Menge von 0,0001-0,5 Gew.-%, bevorzugt 0,001-0,01 Gew.-% enthalten sein, jeweils bezogen auf das Gesamtgewicht der Zusammensetzung.

In der erfindungsgemäßen Zusammensetzung kann Silicium (Kieselsäuren) in einer Menge von 0,01-10,0 Gew.-%, bevorzugt 0,1-5,0 Gew.-% enthalten sein, jeweils bezogen auf das Gesamtgewicht der Zusammensetzung.

Taurin oder 2-Aminoethansulfonsäure wirkt erfindungsgemäß als Antioxidans ebenfalls weiter stabilisierend auf die Haut und Haare und insbesondere die Haarwurzel. In der erfindungsgemäßen Zusammensetzung kann Taurin in einer Menge von 0,001 Gew.-% bis 10,0 Gew.-%, bevorzugt 0,005 Gew.-% bis 7,50 Gew.-% enthalten sein, jeweils bezogen auf das Gesamtgewicht der Zusammensetzung.

Tocopherylacetat zeigt antioxidative Eigenschaften und wirkt erfindungsgemäß weiter stabilisierend auf die Haut und die Haare und insbesondere die Haarwurzel. In der erfindungsgemäßen Zusammensetzung kann Tocopherylacetat in einer Menge von 0,001 Gew.-% bis 10,0 Gew.-%, bevorzugt 0,005 Gew.-% bis 7,50 Gew.-% enthalten sein, jeweils bezogen auf das Gesamtgewicht der Zusammensetzung.

In der erfindungsgemäßen Zusammensetzung kann Tomatenextrakt in einer Menge von 0,1-20,0 Gew.-%, bevorzugt 1-15 Gew.-% enthalten sein, jeweils bezogen auf das Gesamtgewicht der Zusammensetzung.

Ubichinon-10 (Q10 oder Coenzym Q10) ist ein Chinon-Derivat. Das zum Ubichinon-Pool gehörende Q10 gilt als Antioxidans und wirkt erfindungsgemäß stabilisierend auf die Haut und Haare und insbesondere die Haarwurzel. In der erfindungsgemäßen Zusammensetzung kann Ubichinon-10 in einer Menge von 0,001 Gew.-% bis 10,0 Gew.-%, bevorzugt 0,005 Gew.-% bis 7,50 Gew.-% enthalten sein, jeweils bezogen auf das Gesamtgewicht der Zusammensetzung.

In der erfindungsgemäßen Zusammensetzung kann Vitamin B1 (Thiamin) in einer Menge von 0,01-5,0 Gew.-%, bevorzugt 0,05-1,0 Gew.-% enthalten sein, jeweils bezogen auf das Gesamtgewicht der Zusammensetzung.

In der erfindungsgemäßen Zusammensetzung kann Vitamin B2 (Riboflavin) in einer Menge von 0,01-5,0 Gew.-%, bevorzugt 0,05-1,0 Gew.-% enthalten sein, jeweils bezogen auf das Gesamtgewicht der Zusammensetzung.

In der erfindungsgemäßen Zusammensetzung kann Vitamin B6 in einer Menge von 0,01-5,0 Gew.-%, bevorzugt 0,05-0,5 Gew.-% enthalten sein, jeweils bezogen auf das Gesamtgewicht der Zusammensetzung.

In der erfindungsgemäßen Zusammensetzung kann Vitamin B12 in einer Menge von 0,00001-0,01 Gew.-%, bevorzugt 0,00005-0,0005 Gew.-% enthalten sein, jeweils bezogen auf das Gesamtgewicht der Zusammensetzung.

In der erfindungsgemäßen Zusammensetzung kann Vitamin C in einer Menge von 0,1-20,0 Gew.-%, bevorzugt 1-15 Gew.-% enthalten sein, jeweils bezogen auf das Gesamtgewicht der Zusammensetzung.

In der erfindungsgemäßen Zusammensetzung kann Vitamin E in einer Menge von 0,01-10,0 Gew.-%, bevorzugt 0,1-5,0 Gew.-% enthalten sein, jeweils bezogen auf das Gesamtgewicht der Zusammensetzung.

Zink kann als Substanz mit antimikrobieller Wirkung der erfindungsgemäßen Zusammensetzung in einer Menge von 0,01-30,0 Gew.-%, bevorzugt 0,1-5,0 Gew.-% zugesetzt werden, jeweils bezogen auf das Gesamtgewicht der Zusammensetzung.

Erfindungsgemäß wird die Zusammensetzung oral und/oder topisch angewendet. Unter einer oralen Anwendung ist eine innerliche Anwendung zu verstehen, insbesondere durch Tabletten oder Kapseln. "Topisch" beschreibt eine äußerliche, in der Regel äußerlich örtliche, Anwendung.

In einer bevorzugten Ausführungsform ist die Zusammensetzung gemäß der vorliegenden Erfindung ein Nahrungsergänzungsmittel, insbesondere ein Nahrungsergänzungsmittel zur Förderung des Stoffwechsels der Haare und/oder der Haut und/oder zur Förderung des Haarwachstums und/oder zur Behandlung und/oder Vorbeugung von Haarausfall.

Erfindungsgemäße Zusammensetzungen, insbesondere für Nahrungsergänzungsmittel, sind bevorzugt in Form von Kapseln, Lutschtabletten, Tabletten, Pillen und anderen ähnlichen Darreichungsformen, Pulverbeuteln, Flüssigampullen, Flaschen mit Tropfeinsatz und ähnlichen Darreichungsformen von Flüssigkeiten und Pulvern zur Aufnahme des Produktes.

In einer besonders bevorzugten Ausführungsform umfasst die erfindungsgemäße Zusammensetzung a) Calciumhypophosphit und b) Dimethylglycin und/oder ein Salz von Dimethylglycin, insbesondere Natrium-dimethylglycinat oder Dimethylglycin-hydrochlorid, und ist zur oralen Anwendung ausgestaltet.

In einer alternativen bevorzugten Ausführungsform umfasst die erfindungsgemäße Zusammensetzung a) Calciumhypophosphit und b) Dimethylglycin und/oder ein Salz von Dimethylglycin, insbesondere Natrium-dimethylglycinat oder Dimethylglycin-hydrochlorid, und ist zur topischen Anwendung ausgestaltet.

In einer bevorzugten Ausführungsform umfasst die erfindungsgemäße Zusammensetzung insbesondere a) Calciumhypophosphit und b) Dimethylglycin und/oder ein Salz von Dimethylglycin, insbesondere Natrium-dimethylglycinat oder Dimethylglycin-hydrochlorid, und optional wenigstens einen weiteren Wirkstoff c) wie hierin definiert.

In einer bevorzugten Ausführungsform sind die Bestandteile,
a) Phosphinsäure und/oder wenigstens ein Salz der Phosphinsäure, insbesondere Calciumhypophosphit,
b) Dimethylglycin und/oder ein Salz von Dimethylglycin, insbesondere Natrium-dimethylglycinat oder Dimethylglycin-hydrochlorid,
c) optional wenigstens einen weiteren Wirkstoff wie hierin definiert, sowie optional ein oder mehrere geeignete Trägerstoff(e) (d) und/oder ein oder mehrere Füllstoff(e) (e) umfasst.

Geeignete Trägerstoffe (d) umfassen, ohne darauf beschränkt zu sein, Pflanzenöle, wie beispielsweise Kokusnussöl, Sojaöl, Sojalecithin, Sonnenblumenöl, Olivenöl, Palmöl, Rapsöl und Leinöl; Bienenwachs; Zuckeralkohole, wie beispielsweise Sorbit, Mannit, Isomalt, Maltit, Lactit, Xylit und Erythrit; Monosaccharide wie Dextrose (Glucose), Mannose, Fructose; Disaccharide wie Maltose; und unverdauliche Kohlenhydrate wie beispielsweise Cellulose und Pektine.

Erfindungsgemäß bevorzugt werden ein oder mehrere Pflanzenöl(e) als Trägerstoff in Kapseln eingesetzt. Für Tabletten kommen vorzugsweise ein oder mehrere Zucker oder Zuckeralkohole, vorzugsweise Xylit zum Einsatz.

Geeignete Füllstoffe (e) sind dem Fachmann bekannt. Sie werden insbesondere bei der Herstellung von oralen Verabreichungsformen wie Tabletten oder Kapseln mit geringen Wirkstoffmengen verwendet, um eine für die Herstellung nötige Größe oder Masse der Tablette zu gewährleisten. Bevorzugte Füllstoffe sind Stärke bzw. deren Bestandteile Amylopektin und Amylose und/oder Amylodextrin.

Stärke ist natürlicher Bestandteil von Kartoffeln, Maniok, Getreide (z.B. Weizen, Mais, Reis) sowie Hülsenfrüchten (z.B. Erbsen, Linsen, weiße oder rote Bohnen, Kichererbsen). Erfindungsgemäß ist Mais-, Kartoffel-, Weizen-, Reis- und/oder Hirsestärke insbesondere bevorzugt.

Amylopektin bezeichnet ein Polysaccharid aus α-D-Glucose-Monomeren, die stark verzweigte Strukturen bilden können. Die α-D-Glucose-Monomere sind dabei α-1,4-glycosidisch und an Verzweigungen α-1,6-glycosidisch miteinander verbunden.

Amylose bezeichnet ein Polysaccharid aus α-D-Glucose-Monomeren, die lineare Ketten mit helikaler Struktur bilden. Amylose hat eine molare Masse zwischen 100.000 und 1.000.000 g/mol, entsprechend etwa 1000 α-D-Glucose-Monomeren (bei Getreidestärken) bis 4500 α-D-Glucose-Monomeren (bei Kartoffelstärken), die α-1,4-glycosidisch miteinander verbunden sind. Bei hohen molaren Massen treten auch vereinzelte α-1,6-glycosidische Verzweigungen auf.

Amylodextrin ist ein Umwandlungsprodukt der Stärke mittels verdünnter Säuren. Das Amylodextrin (6 C₆H₁₀O₅ + 2 H₂O) ist eine der Stärke isomere Verbindung. Amylodextrin kann auch als Endprodukt der Hydrolyse von Amylopektin durch β-Amylase gewonnen werden.

Die erfindungsgemäße Zusammensetzung kann in jeder zur oralen oder topischen Verabreichung geeigneten Darreichungsform vorliegen. Orale Verabreichungsformen sind bevorzugt. Die Zusammensetzung kann entsprechend in einer flüssigen, festen oder halbfesten Darreichungsform vorliegen.

Flüssige Darreichungsformen umfassen Lösungen, Emulsionen, Suspensionen und Sirup. Soweit die erfindungsgemäße Zusammensetzung flüssig oder halbfest vorliegt weist sie bevorzugt einen pH-Wert zwischen 3,0 und 9,0 auf. Der pH-Wert liegt vorzugsweise im Bereich von 4,0 bis 8,0, insbesondere zwischen 5,0 und 7,0.

Der pH-Wert kann durch pH-Regulatoren eingestellt werden. pH-Regulatoren sind Substanzen, die einen bestimmten pH-Wert-Bereich, vorzugsweise einen Bereich von pH 5,5 bis 8,0, einstellen können. Beispiele für pH-Regulatoren sind Essigsäure, Acetate, Milchsäure, Lactate, Äpfelsäure, Malate, Fumarsäure, Citronensäure, Citrate, Weinsäure, Tartrate, Orthophosphaten, Di-, Tri- und Polyphosphate, Salzsäure, Chloriden, Schwefelsäure, Sulfate, Hydroxiden, Oxide, Adipinsäure, Adipate, Gluconsäure, Gluconate, Phosphorsäure, Calciumcarbonat oder ein Hydrat davon. Ein bevorzugtes Beispiel für einen pH-Regulator, der zugegeben werden kann, wenn ein niedrigerer pH-Wert gewünscht wird, ist Phosphorsäure (H₃PO₄).

Halbfeste Darreichungsformen umfassen beispielsweise Gele, Pasten, Salben und Cremes.

Zu den festen Darreichungsformen zählen Tabletten, Dragees, Kapseln, Granulate und Pulver zum Einnehmen. Feste Darreichungsformen können mit modifizierter Wirkstofffreisetzung (z.B. verlängert oder verzögert) gefertigt werden.

Bevorzugt ist erfindungsgemäß eine Darreichungsform der Zusammensetzung als Tablette verpresst oder in einer Kapsel, z.B. einer Hartgelatinekapsel oder Weichgelatinekapsel.

Die erfindungsgemäße Zusammensetzung kann durch alle dem Fachmann bekannte Verfahren hergestellt werden.

Die vorliegende Erfindung bezieht sich weiterhin auf die Verwendung der erfindungsgemäßen Zusammensetzung zur Verbesserung des Zustands der Haare und/oder der Haut und/oder zur Förderung des Stoffwechsels der Haare und/oder der Haut und/oder zur Förderung des Haarwachstums und/oder zur Behandlung und/oder Vorbeugung von Haarausfall.

Dabei umfasst der Begriff Haarausfall Alopecia areata (kreisrunden Haarausfall), Alopecia androgenetica (erblich bedingten Haarausfall) bei Frauen und Männern, diffuse Alopezie (diffusen Haarausfall), altersbedingten Haarausfall (senescent Alopecia) und durch Chemotherapie-bedingten Haarausfall.

Besonders bevorzugt ist der zu behandelnde Haarausfall erblich bedingter Haarausfall (Alopecia androgenetica).

Ebenso besonders bevorzugt ist der zu behandelnde Haarausfall altersbedingter Haarausfall (senescent Alopecia).

Die Verwendung ist insbesondere eine kosmetische oder medizinische Verwendung.

Die erfindungsgemäße Verwendung führte dabei überraschenderweise nicht nur zu einer Verringerung des täglichen Haarausfalls, sondern darüber hinaus auch zu einer Zunahme der Haardichte und zu positiven Effekten auf die Haarwurzelaktivität im Vergleich zu einer Kontrollgruppe, wobei die Haardichte sowie die Haarwurzelaktivität durch etablierte Standardverfahren aus dem Bereich Dermatologie vor und nach der Einnahme bestimmt wurden (siehe u.a. Serrano-Falcón C, Fernändez-Pugnaire MA, Serrano-Ortega S. Hair and scalp evaluation: the trichogram.).

Ein weiterer Aspekt der vorliegenden Erfindung betrifft einen Kit umfassend
a) Dimethylglycin und/oder ein Salz von Dimethylglycin, insbesondere Dimethylglycin,
b) Phosphinsäure und/oder wenigstens ein Salz der Phosphinsäure, insbesondere Calciumhypophosphit,
wobei a) und b) in getrennten Verabreichungsformen vorliegen.

A) und b) können zur oralen und/oder topischen Verabreichung vorgesehen sein. Beide Wirkstoffe können beispielsweise in Form einer Tablette verabreicht werden. Es ist aber auch möglich einen Wirkstoff als Tablette und den entsprechend anderen Wirkstoff topisch, z.B. in Form einer Creme, Lotion, Suspension oder Shampoo-Grundlage zu verabreichen. Die beiden Verabreichungsformen können gleichzeitig oder zeitlich versetzt voneinander eingenommen werden. So ist es beispielsweise möglich eine Tablette umfassend einen der Wirkstoffe am Morgen und eine Tablette umfassend den entsprechend anderen Wirkstoff am Abend einzunehmen. Genauso kann eine Lotion umfassend einem Wirkstoff am Morgen topisch angewendet werden und eine Tablette umfassend den entsprechend anderen Wirkstoff am Abend eingenommen werden.

Anhand der nachfolgenden Zusammensetzungen soll die Erfindung verdeutlicht werden, ohne sie jedoch auf die speziellen Beispiele einschränken zu wollen.

### Beispiele

Die folgenden Zusammensetzungen wurden durch für den Fachmann bekannte Maßnahmen hergestellt. Die Menge der Komponenten wurde jeweils so gewählt, dass ihr Gewichtsanteil in der Vormischung den angegebenen Gewichtsanteilen entspricht.

### Beispiel 1

Zusammensetzung in Form einer Tablette, welche die folgenden Komponenten enthält:

| Nährstoff | Einzeldosis [mg] | Gew.-% |
|---|---|---|
| Vitamin C | 80 | 8,6 |
| Vitamin E (α-Tocopherol) | 5 | 0,5 |
| Vitamin B2 | 1 | 0,1 |
| Vitamin B12 | 0,002 | 0,0002 |
| Zink | 15 | 1,6 |
| Panthothensäure | 10 | 1,1 |
| Biotin/Vitamin H | 0,1 | 0,01 |
| Folsäure | 0,2 | 0,02 |
| Silicium | 10 | 1,1 |
| L-Cystein | 5 | 0,5 |
| Hirseextrakt | 300 | 32,4 |
| L-Lysin | 100 | 10,8 |
| Na-*N*,*N*-Dimethylglycinat | 200 | 21,6 |
| Calciumhypophosphit | 200 | 21,6 |

Die Tablette kann ferner übliche Trägerstoffe, wie Zuckeralkohol, und/oder Füllstoffe enthalten.

### Beispiel 2

Zusammensetzung in Form einer Tablette, welche die folgenden Komponenten enthält:

| Nährstoff | Einzeldosis [mg] | Gew.-% |
|---|---|---|
| Vitamin C | 80 | 8,2 |
| Vitamin E (α-Tocopherol) | 5 | 0,5 |
| Vitamin B2 | 1 | 0,1 |
| Vitamin B12 | 0,002 | 0,0002 |
| Zink | 15 | 1,5 |
| Panthothensäure | 10 | 1,0 |
| Biotin/Vitamin H | 0,1 | 0,010 |
| Folsäure | 0,2 | 0,02 |
| Silicium | 10 | 1,0 |
| L-Cystein | 5 | 0,5 |
| Hirseextrakt | 300 | 30,7 |
| L-Lysin | 100 | 10,2 |
| Na-*N*,*N*-Dimethylglycinat | 300 | 30,7 |
| Calciumhypophosphit | 150 | 15,4 |

Die Tablette kann ferner übliche Trägerstoffe, wie Zuckeralkohol, und/oder Füllstoffe enthalten.

### Beispiel 3

Zusammensetzung in Form einer Kapsel, welche die folgenden Komponenten enthält:

| Nährstoff | Einzeldosis [mg] | Gew.-% |
|---|---|---|
| Vitamin C | 100 | 21,6 |
| Vitamin E (α-Tocopherol) | 10 | 2,2 |
| Niacin | 18 | 3,9 |
| Zink | 5 | 1,1 |
| ß-Carotin/Provitamin A | 2 | 0,4 |
| Vitamin B6 | 1,8 | 0,4 |
| Vitamin B2 (Riboflavin) | 1 | 0,2 |
| Vitamin B1 (Thiamin) | 1,2 | 0,3 |
| Folsäure | 0,3 | 0,1 |
| Biotin | 0,12 | 0,03 |
| Selen | 0,03 | 0,01 |
| Vitamin B12 | 0,001 | 0,0002 |
| *N,N*-Dimethylglycin - hydrochlorid | 162 | 35,0 |
| Calciumhypophosphit | 162 | 35,0 |

Die Kapsel kann ferner übliche Trägerstoffe, wie Pflanzenöl, und/oder Füllstoffe enthalten.

### Beispiel 4

Zusammensetzung in Form einer Kapsel, welche die folgenden Komponenten enthält:

| Nährstoff | Einzeldosis [mg] | Gew.-% |
|---|---|---|
| Biotin | 1 | 0,03 |
| Vitamin C | 100 | 3,0 |
| Vitamin E (α-Tocopherol) | 6 | 0,2 |
| Zink | 15 | 0,4 |
| Selen | 0,2 | 0,01 |
| Eisen | 8 | 0,2 |
| Sägepalme-Extrakt | 400 | 11,9 |
| Schachtelhalm-Extrakt | 20 | 0,6 |
| Amla Extrakt | 600 | 17,8 |
| Kürbiskernöl | 300 | 8,9 |
| Procyanidin | 200 | 5,9 |
| Grüner Tee Extrakt | 100 | 3,0 |
| Rosmarinextrakt | 2 | 0,1 |
| Curcumin | 300 | 8,9 |
| Piperin | 5 | 0,1 |
| Granatapfelextrakt | 400 | 11,9 |
| Hirseöl | 300 | 8,9 |
| L-Cystein | 10 | 0,3 |
| L-Lysin | 200 | 5,9 |
| *N,N*-Dimethylglycin - hydrochlorid | 100 | 3,0 |
| Calciumhypophoshit | 300 | 8,9 |

Die Kapsel kann ferner übliche Trägerstoffe, wie Pflanzenöl, und/oder Füllstoffe enthalten.

### Beispiel 5

Zusammensetzung in Form eines Shampoos, welches die folgenden Komponenten enthält:

| | |
|---|---|
| Natriummyrethsulfat | 4,0 Gew.% |
| Natriumlaurethsulfat | 4,0 Gew.% |
| Dinatriumlaurethsulfosuccinat | 3,0 Gew.% |
| Tocopherylacetat | 0,3 Gew.% |
| Zitronensäure | 1,0 Gew.% |
| Calciumhypophosphit | 0,5 Gew.% |
| Na-*N*,*N*-Dimethylglycinat | 1,0 Gew.% |
| Parfum | 0,3 Gew.% |
| Kaliumsorbat | 0,2 Gew.% |
| Natriumbenzoat | 0,1 Gew.% |
| Wasser | ad 100 Gew.% |

### Beispiel 6

Zusammensetzung in Form eines Haarwassers, welches die folgenden Komponenten enthält:

| | |
|---|---|
| Alkohol denat. | 30,0 Gew.% |
| Amodimethicon | 0,5 Gew.% |
| Panthenol | 0,5 Gew.% |
| Calciumhypophosphit | 0,25 Gew.% |
| *N,N*-Dimethylglycin -hydrochlorid | 0,25 Gew.% |
| Bisabolol | 0,2 Gew.% |
| PEG-25 PABA | 0,5 Gew.% |
| PEG-16 (hydriertes Rizinusöl) | 0,5 Gew.% |
| Parfum | 0,3 Gew.% |
| Menthol | 0,3 Gew.% |
| Milchsäure | 0,1 Gew.% |
| Wasser | ad 100 Gew.% |

### Beispiel 7

Zusammensetzung in Form einer Emulsion, welche die folgenden Komponenten enthält:

| | |
|---|---|
| Zink-Stearat | 1,25 Gew.% |
| Magnesium-Sulfat | 0, 5 Gew.% |
| Sorbitan Isostearate & Polyglyceryl-3 Polyricinoleate | 4,5 Gew.% |
| Hexyldecanol & Hexyldecyllaurat | 25,0 Gew.% |
| Glycerin | 2,0 Gew.% |
| Zitronensäure | 0,4 Gew.% |
| Calciumhypophosphit | 0,1 Gew.% |
| *N,N*-Dimethylglycin -hydrochlorid | 0,4 Gew.% |
| Parfum | 0,2 Gew.% |
| Konservierungsmittel | q.s. |
| Wasser | ad 100 Gew.% |

### Beispiel 8

Zusammensetzungen in Form eines Kits, welches die folgenden Verabreichungsformen und Komponenten enthält:

Kit-Komponente 1: Shampoo, welches die folgenden Komponenten enthält:

| | |
|---|---|
| Natriummyrethsulfat | 4,0 Gew.% |
| Natriumlaurethsulfat | 4,0 Gew.% |
| Dinatriumlaurethsulfosuccinat | 3,0 Gew.% |
| Tocopherylacetat | 0,3 Gew.% |
| Zitronensäure | 1,0 Gew.% |
| Na-*N*,*N*-Dimethylglycinat | 1,0 Gew.% |
| Parfum | 0,3 Gew.% |
| Kaliumsorbat | 0,2 Gew.% |
| Natriumbenzoat | 0,1 Gew.% |
| Wasser | ad 100 Gew.% |

Kit-Komponente 2: Zusammensetzung in Form einer Kapsel, welche die folgenden Komponenten enthält:

| Nährstoff | Einzeldosis [mg] | Gew.-% |
|---|---|---|
| Vitamin C | 100 | 29,5 |
| Vitamin E (α-Tocopherol) | 10 | 2,9 |
| Niacin | 18 | 5,3 |
| Zink | 5 | 1,5 |
| β-Carotin/Provitamin A | 2 | 0,6 |
| Vitamin B6 | 1,8 | 0,5 |
| Vitamin B2 (Riboflavin) | 1 | 0,3 |
| Vitamin B1 (Thiamin) | 1,2 | 0,4 |
| Folsäure | 0,3 | 0,1 |
| Biotin | 0,12 | 0,0354 |
| Selen | 0,03 | 0,0088 |
| Vitamin B12 | 0,001 | 0,0003 |
| Calciumhypophosphit | 200 | 58,9 |

Die Kapsel kann ferner übliche Trägerstoffe, wie Pflanzenöl, und/oder Füllstoffe enthalten.

### Beispiel 9

Zusammensetzungen in Form eines Kits, welches die folgenden Verabreichungsformen und Komponenten enthält:

Kit-Komponente 1: Shampoo, welches die folgenden Komponenten enthält:

| | |
|---|---|
| Natriummyrethsulfat | 4,0 Gew.% |
| Natriumlaurethsulfat | 4,0 Gew.% |
| Dinatriumlaurethsulfosuccinat | 3,0 Gew.% |
| Tocopherylacetat | 0,3 Gew.% |
| Zitronensäure | 0,2 Gew.% |
| Calciumhypophosphit | 0,2 Gew.% |
| Parfum | 0,3 Gew.% |
| Kaliumsorbat | 0,2 Gew.% |
| Natriumbenzoat | 0,1 Gew.% |
| Wasser | ad 100 Gew.% |

Kit-Komponente 2: Zusammensetzung in Form einer Kapsel, welche die folgenden Komponenten enthält:

| Nährstoff | Einzeldosis [mg] | Gew.-% |
|---|---|---|
| Vitamin C | 100 | 33,2 |
| Vitamin E (α-Tocopherol) | 10 | 3,3 |
| Niacin | 18 | 6,0 |
| Zink | 5 | 1,7 |
| β-Carotin/Provitamin A | 2 | 0,7 |
| Vitamin B6 | 1,8 | 0,6 |
| Vitamin B2 (Riboflavin) | 1 | 0,3 |
| Vitamin B1 (Thiamin) | 1,2 | 0,4 |
| Folsäure | 0,3 | 0,1 |
| Biotin | 0,12 | 0,0398 |
| Selen | 0,03 | 0,0100 |
| Vitamin B12 | 0,001 | 0,0003 |
| *N,N*-Dimethylglycin - hydrochlorid | 162 | 53,7 |

Die Kapsel kann ferner übliche Trägerstoffe, wie Pflanzenöl, und/oder Füllstoffe enthalten.

### Beispiel 10

Zusammensetzung in Form einer Hautpflegecreme, welche die folgenden Komponenten enthält:

| Wasser | ad 100 |
|---|---|
| Glycerin | 5 |
| Cetylalkohol | 2 |
| Glycerylstearat | 1,5 |
| Squalan | 1,5 |
| Cetearylethylhexanoat | 1 |
| Octyldodecanol | 3 |
| Oleylerucat | 1,5 |
| Avocadoöl | 0,5 |
| Ethyllinoleat | 1,5 |
| PEG(40)stearat | 2 |
| Phenoxyethanol | 0,5 |
| Tocopherol | 0,5 |
| Calciumhypophosphit | 1 |
| Phytosterole | 0,5 |
| Pflanzliches Öl | 0,5 |
| Sorbitantristearat | 0,5 |
| Olivenöl | 0,5 |
| Natrium-N,N-dimethylglycinat | 1 |

Die Mengenangaben entsprechen jeweils dem Gewichtsanteil in % an der fertigen Zusammensetzung.

### Beispiel 11:

Zusammensetzung in Form einer Hautpflegecreme, welche die folgenden Komponenten enthält:

| Wasser | ad 100 |
|---|---|
| Glycerin | 5 |
| Cetylalkohol | 2 |
| Glycerylstearat | 1,5 |
| Squalan | 1,5 |
| Cetearylethylhexanoat | 1 |
| Octyldodecanol | 3 |
| Oleylerucat | 1,5 |
| Avocadoöl | 0,5 |
| Ethyllinoleat | 1,5 |
| PEG(40)stearat | 2 |
| Phenoxyethanol | 0,5 |
| Tocopherol | 0,5 |
| Calciumhypophosphit | 1 |
| Phytosterole | 0,5 |
| Pflanzliches Öl | 0,5 |
| Sorbitantristearat | 0,5 |
| Olivenöl | 0,5 |
| Natrium-N,N-dimethylglycinat | 1 |

Die Mengenangaben entsprechen jeweils dem Gewichtsanteil in % an der fertigen Zusammensetzung.

### Beispiel 13:

Zusammensetzung in Form einer Hautpflegecreme, welche die folgenden Komponenten enthält:

| Wasser | ad 100 |
|---|---|
| Decyloleat | 7 |
| Isopropylmyristat | 6 |
| Hexyldecanol | 4 |
| Hexyldecyllaurat | 3,5 |
| Distelöl | 5 |
| Glycerin | 4 |
| Sorbitan-Isostearat | 2 |
| Polyglycerin-3 Polyricinoleat | 3 |
| Cera Alba | 2,5 |
| Zinkstearat | 1 |
| Benzylalkohol | 0,5 |
| Magnesiumsulfat | 0,4 |
| Echinacea Purpurea Wurzelextrakt | 0,1 |
| Tocopherol | 0,5 |
| Calciumhypophosphit | 2 |
| Natrium-N,N-dimethylglycinat | 1,7 |

Die Mengenangaben entsprechen jeweils dem Gewichtsanteil in % an der fertigen Zusammensetzung.

### Beispiel 14:

Zusammensetzung in Form einer Hautpflegecreme, welche die folgenden Komponenten enthält:

| | |
|---|---|
| Caprylsäure-/Caprinsäuretriglycerid | 25 |
| Cocoglyceride | 25 |
| Wasser | ad 100 |
| Octyldodecylmyristat | 9 |
| Oleylerucat | 8 |
| Glycerylstearat | 5 |
| Glycerylbehenat | 5 |
| Jojoba-Ester | 5 |
| Sonnenblumenwachs | 1,5 |
| Hydriertes Rizinusöl | 3 |
| Benzylalkohol | 1 |
| Pentylenglykol | 0,5 |
| Butylenglykol | 0,5 |
| Mimosenwachs | 0,8 |
| Polyglycerin-3 | 0,25 |
| Bisabolol | 0,4 |
| Octenidindihydrochlorid | 0,05 |
| Hydroxyphenylpropamidobenzoesäure | 0,1 |
| Natrium-N, N-Dimethylglycinat | 1,5 |
| Calciumhypophosphit | 1,7 |

Die Mengenangaben entsprechen jeweils dem Gewichtsanteil in % an der fertigen Zusammensetzung.

### Referenzbeispiel 1

Zusammensetzung in Form einer Tablette (ohne Na-Dimethylglycinat und Calciumhypophoshit), welche die folgenden Komponenten enthält:

| Nährstoff | Einzeldosis [mg] | Gew.-% |
|---|---|---|
| Vitamin C | 80 | 15,2 |
| Vitamin E (α-Tocopherol) | 5 | 1,0 |
| Vitamin B2 | 1 | 0,2 |
| Vitamin B12 | 0,002 | 0,0 |
| Zink | 15 | 2,9 |
| Panthothensäure | 10 | 1,9 |
| Biotin/Vitamin H | 0,1 | 0,02 |
| Folsäure | 0,2 | 0,04 |
| Silicium | 10 | 1,9 |
| L-Cystein | 5 | 1,0 |
| Hirseextrakt | 300 | 57,0 |
| L-Lysin | 100 | 19,0 |

Die Tablette kann ferner übliche Trägerstoffe, wie Zuckeralkohol, und/oder Füllstoffe enthalten.

### Studiendesign und -ergebnisse

Im Rahmen einer *in vitro* Untersuchung wurde die Wirkung von Calciumhypophoshit bzw. Na-Dimethylglycinat sowie deren Kombination im Zellkulturmodell mit humanen hornbildenden Keratinozyten-Zellen untersucht. Hierfür wurden HaCaT-Zellen für 1, 3, 5, und 7 Tage in DMEM-Medium (inklusive 2.5% fötalem Kälberserum und einem Antibiotika-Antimykotika-Mix) kultiviert und u.a. die Viabilität und Proliferation der Zellen durch geeignete Messmethoden bestimmt sowie die Bildung der für das Zellwachstum relevanten Signalmoleküle gemessen.

Es hat sich gezeigt, dass insbesondere die Kombination von Calciumhypophosphit mit Na-Dimethylglycinat die für das Wachstum relevanten Parameter der HaCaT-Zellen positiv beeinflußt und die Bildung der Wachstumsfaktoren (beispielsweise VEGF und intrazelluläres Calcium) signifikant gesteigert wurde gegenüber der Behandlung von HaCaT-Zellen mit Calciumhypophoshit oder Na-Dimethylglycinat alleine.

Die erfindungsgemäße Zusammensetzung aus Beispiel 1 (Verum-Zusammensetzung mit Natrium-Dimethylglycinat und Calciumhypophospit) und die Zusammensetzung aus Referenzbeispiel 1 (Placebo-Zusammensetzung ohne Natrium-Dimethylglycinat und Calciumhypophospit) wurden im Rahmen einer randomisierten, kontrollierten und verblindeten Anwendungsstudie an einem Probandenkollektiv von insgesamt 60 Männern und Frauen für 6 Monate eingenommen.

Die Probanden wurden zur subjektiven Bewertung des Rückgangs ihres Haarausfalls nach Beendigung der Einnahme befragt, wobei diese Ergebnisse mit Hilfe von Fragebögen festgehalten wurden. Gleichzeitig wurde die Haardichte sowie die Haarwurzelaktivität durch etablierte Standardverfahren aus dem Bereich Dermatologie vor und nach der Einnahme bestimmt (siehe u.a. Serrano-Falcön C, Fernändez-Pugnaire MA, Serrano-Ortega S. Hair and scalp evaluation: the trichogram.).

Während bzw. nach der oralen Einnahme der erfindungsgemäßen Zusammensetzung aus Beispiel 1 gaben mehr Probanden und Probandinnen an, dass weniger Haare ausgefallen sind im Vergleich zur Kontrollgruppe unter oraler Einnahme der Placebo-Zusammensetzung. Im Rahmen der objektiven Messungen zeigte sich, dass unter oraler Einnahme der erfindungsgemäßen Zusammensetzung aus Beispiel 1 eine Zunahme der Haardichte und positive Effekte auf Haarwurzelaktivität nachgewiesen wurden im Vergleich zur Kontrollgruppe.

Die folgenden Punkte sind Gegenstand der Erfindung:
1. Zusammensetzung umfassend
   (a) Phosphinsäure und/oder wenigstens ein Salz der Phosphinsäure, und
   (b) Dimethylglycin und/oder ein Salz von Dimethylglycin.
2. Zusammensetzung gemäß einem der vorangegangenen Punkte, wobei das Salz der Phosphinsäure (a) zweiwertig ist.
3. Zusammensetzung gemäß einem der vorangegangenen Punkte, wobei es sich bei dem Salz der Phosphinsäure (a) um das Calciumsalz handelt, d.h. Calciumhypophosphit.
4. Zusammensetzung gemäß einem der vorangegangenen Punkte, wobei die Zusammensetzung 0,0001 bis 35,0 Gewichtsprozent (Gew.-%), bevorzugt 0,001 bis 25,0 Gew.-%, stärker bevorzugt 0,01 bis 15,0 Gew.-%, noch stärker bevorzugt 0,1 bis 10,0 Gew.-%, und besonders bevorzugt 0,1 bis 5,0 Gew.-% Phosphinsäure und/oder ein Salz der Phosphinsäure, insbesondere Calciumhypophosphit, enthält.
5. Zusammensetzung nach einem der vorangehenden Punkte, wobei die Zusammensetzung 0,0001 bis 35,0 Gewichtsprozent (Gew.-%), bevorzugt 0,001 bis 25,0 Gew.-%, stärker bevorzugt 0,01 bis 15,0 Gew.-%, noch stärker bevorzugt 0,1 bis 10,0 Gew.-%, und besonders bevorzugt 0,1 bis 5,0 Gew.-% Dimethylglycin und/oder ein Salz von Dimethylglycin enthält.
6. Zusammensetzung gemäß einem der vorangegangenen Punkte, wobei die Zusammensetzung Dimethylglycin und/oder ein Salz davon sowie Phospinsäure und/oder ein Salz davon in einem molaren Verhältnis von 1:10 bis 10:1, bevorzugt 1:5 bis 5:1 und besonders bevorzugt 1:2 bis 2:1 enthält.
7. Zusammensetzung nach einem der vorangegangenen Punkte, wobei die Zusammensetzung ferner umfasst mindestens einen weiteren Wirkstoff c), ausgewählt aus Amla-Extrakt, β-Carotin, Biotin, Carnitin, Curcumin, Echinacea, Ectoin, Eisen, Folsäure, Granatapfel-Extrakt, Grüntee-Extrakt, Hirseextrakt, Hirseöl, Kürbiskernöl, Kreatin, L-Cystein, L-Lysin, Niacin, Niacinamid, Pantolacton, Panthothensäure, Piperin, Procyanidin, Rosmarin-Extrakt, Sägepalme-Extrakt, Schachtelhalm-Extrakt, Selen, Silicium, Taurin, Tocopherylacetat, Tomatenextrakt, Ubichinon-10, Vitamin B1, Vitamin B2, Vitamin B6, Vitamin B12, Vitamin C, Vitamin E, Zink und Kombinationen hiervon.
8. Zusammensetzung gemäß einem der vorangegangenen Punkte, wobei die Zusammensetzung zur oralen und/oder topischen Anwendung ausgestaltet ist.
9. Zusammensetzung gemäß einem der vorangegangenen Punkte, wobei die Zusammensetzung zur oralen Anwendung ausgestaltet ist.
10. Zusammensetzung gemäß einem der vorangegangenen Punkte, wobei es sich bei dem Salz der Phosphinsäure (A) um das Calciumsalz handelt, d.h. Calciumhypophosphit, und wobei die Zusammensetzung zur oralen Anwendung ausgestaltet ist.
11. Verwendung der Zusammensetzung nach einem der vorangegangenen Punkte als Nahrungsergänzungsmittel, insbesondere zur Förderung des Stoffwechsels der Haare und/oder der Haut und/oder zur Förderung des Haarwachstums und/oder zur Behandlung und/oder Vorbeugung von Haarausfall ist.
12. Verwendung der Zusammensetzung nach einem der vorangegangenen Punkte zur Verbesserung des Zustands der Haare und/oder der Haut und/oder zur Förderung des Stoffwechsels der Haare und/oder der Haut und/oder zur Förderung des Haarwachstums und/oder zur Behandlung und/oder Vorbeugung von Haarausfall.
13. Verwendung nach Punkt 12, wobei der Haarausfall ausgewählt ist aus Alopecia areata (kreisrundem Haarausfall), Alopecia androgenetica (erblich bedingter Haarausfall) bei Frauen und Männern, diffuser Alopezie (diffusem Haarausfall), altersbedingtem Haarausfall (senescent Alopecia) und durch Chemotherapie bedingtem Haarausfall.
14. Verwendung nach Punkt 12 oder 13, wobei die Verwendung eine medizinische oder kosmetische Verwendung ist.
15. Kit umfassend
   a) Dimethylglycin und/oder ein Salz von Dimethylglycin, insbesondere Dimethylglycin,
   b) Phosphinsäure und/oder wenigstens ein Salz der Phosphinsäure, insbesondere Calciumhypophosphit,
   wobei a) und b) in getrennten Verabreichungsformen vorliegen.

## Patentansprüche

1. Topische Zusammensetzung umfassend
(a) Phosphinsäure und/oder wenigstens ein Salz der Phosphinsäure, und
(b) Dimethylglycin und/oder ein Salz von Dimethylglycin.

2. Zusammensetzung gemäß einem der vorangegangenen Ansprüche, wobei das Salz der Phosphinsäure (a) zweiwertig ist.

3. Zusammensetzung gemäß einem der vorangegangenen Ansprüche, wobei es sich bei dem Salz der Phosphinsäure (a) um das Calciumsalz handelt, d.h. Calciumhypophosphit.

4. Zusammensetzung gemäß einem der vorangegangenen Ansprüche, wobei die Zusammensetzung 0,0001 bis 35,0 Gewichtsprozent (Gew.-%), bevorzugt 0,001 bis 25,0 Gew.-%, stärker bevorzugt 0,01 bis 15,0 Gew.-%, noch stärker bevorzugt 0,1 bis 10,0 Gew.-%, und besonders bevorzugt 0,1 bis 5,0 Gew.-% Phosphinsäure und/oder ein Salz der Phosphinsäure, insbesondere Calciumhypophosphit, enthält.

5. Zusammensetzung nach einem der vorangehenden Ansprüche, wobei die Zusammensetzung 0,0001 bis 35,0 Gewichtsprozent (Gew.-%), bevorzugt 0,001 bis 25,0 Gew.-%, stärker bevorzugt 0,01 bis 15,0 Gew.-%, noch stärker bevorzugt 0,1 bis 10,0 Gew.-%, und besonders bevorzugt 0,1 bis 5,0 Gew.-% Dimethylglycin und/oder ein Salz von Dimethylglycin enthält.

6. Zusammensetzung gemäß einem der vorangegangenen Ansprüche, wobei die Zusammensetzung Dimethylglycin und/oder ein Salz davon sowie Phospinsäure und/oder ein Salz davon in einem molaren Verhältnis von 1:10 bis 10:1, bevorzugt 1:5 bis 5:1 und besonders bevorzugt 1:2 bis 2:1 enthält.

7. Zusammensetzung nach einem der vorangegangenen Ansprüche, wobei die Zusammensetzung ferner umfasst
mindestens einen weiteren Wirkstoff c), ausgewählt aus Amla-Extrakt, β-Carotin, Biotin, Carnitin, Curcumin, Echinacea, Ectoin, Eisen, Folsäure, Granatapfel-Extrakt, Grüntee-Extrakt, Hirseextrakt, Hirseöl, Kürbiskernöl, Kreatin, L-Cystein, L-Lysin, Niacin, Niacinamid, Pantolacton, Panthothensäure, Piperin, Procyanidin, Rosmarin-Extrakt, Sägepalme-Extrakt, Schachtelhalm-Extrakt, Selen, Silicium, Taurin, Tocopherylacetat, Tomatenextrakt, Ubichinon-10, Vitamin B1, Vitamin B2, Vitamin B6, Vitamin B12, Vitamin C, Vitamin E, Zink und Kombinationen hiervon.

8. Zusammensetzung gemäß einem der vorangegangenen Ansprüche, wobei es sich bei dem Salz der Phosphinsäure (A) um das Calciumsalz handelt, d.h. Calciumhypophosphit, und wobei die Zusammensetzung zur topischen Anwendung ausgestaltet ist.

9. Verwendung der Zusammensetzung nach einem der vorangegangenen Ansprüche zur Verbesserung des Zustands der Haare und/oder der Haut und/oder zur Förderung des Stoffwechsels der Haare und/oder der Haut und/oder zur Förderung des Haarwachstums und/oder zur Behandlung und/oder Vorbeugung von Haarausfall.

10. Verwendung nach Anspruch 9, wobei der Haarausfall ausgewählt ist aus Alopecia areata (kreisrundem Haarausfall), Alopecia androgenetica (erblich bedingter Haarausfall) bei Frauen und Männern, diffuser Alopezie (diffusem Haarausfall), altersbedingtem Haarausfall (senescent Alopecia) und durch Chemotherapie bedingtem Haarausfall.

11. Verwendung nach Anspruch 9 oder 10, wobei die Verwendung eine medizinische oder kosmetische Verwendung ist.

12. Kit umfassend
a) Dimethylglycin und/oder ein Salz von Dimethylglycin, insbesondere Dimethylglycin,
b) Phosphinsäure und/oder wenigstens ein Salz der Phosphinsäure, insbesondere Calciumhypophosphit,
wobei a) und b) in getrennten Verabreichungsformen vorliegen, wobei a) und/oder b) zur topischen Verabreichung vorgesehen sind
